# EUROPEAN PATENT APPLICATION

(11) **EP 0 643 977 A1**
(43) Date of publication of application: **22.03.1995**
(21) Application number: 94907573.3
(22) Date of filing: 21.02.1994
(51) Int. Cl.: A61M 5/50

(54) **NEW DISPOSABLE HYPODERMIC SYRINGE**

(30) Priority: 03.03.1993 ES 9300715 U
(71) Applicant: NEW APPLICATION IN PLASTIC, S.L., E-08820 El Prat de Llobregat (ES)
(72) Inventor: RIBAS UDARAUZ, Montserrat, E-08820 El Prat de Llobregat (ES); MORAL POVEDANO, Francisca, E-08820 El Prat de Llobregat (ES)
(74) Representative: Canela Bresco, Arturo
(86) International application number: ES9400018
(87) International publication number: WO9420162

(57) **Abstract**

The new disposable hypodermic syringe (1) has the composite plunger rod formed by two separate parts (2a, 2b) linked to each other by means of a connecting link (5a, 5b), with a cross-sectional articulation point, said link being made of appropriate material, and presenting a cross-sectional perpendicular slot (7) of selected depth and amplitude, said perpendicular slot forming a flexible connecting membrane and acting as a hinge which brakes due to fatigue. On the facing surfaces of the butt ends (3a, 3b) which are spaced apart in a rest position, there are provided projecting crimping means (8, 9) aligned with respect to each other, and any rotation imparted to one of said means is followed by the other means, seaming which disappears when, upon a subsequent traction, the two discs are spaced apart, yet maintained linked by means of the intermediate part.

## Description

The object of the present patent is a new hypodermic syringe of the single-use type.

Single-use hypodermic syringe are known, which in order to complete use thereof, the user of the syringe has to exert a traction or contraction force such that by breaking the safety device, the entire phase of single-use application can be completed, by releasing the locking by means of deliberate excess pressure, thus destroying the aforementioned safety device, and by this means rendering re-use of the syringe impossible.

It is thus understood that rendering the syringe unusable is a result of the action of its user, by deliberately destroying the locking of the piston by means of excess pressure.

These known syringes were developed owing to the need to prevent serious and non-serious contagious illnesses which are frequently transmitted by re-use of a syringe already used by another person.

Thus, pathological infectious germs wich can be contained in the bloodstream of one person are tranferred to the syringe, and despite regular, commonplace disinfection, they can remain inside the syringe and pass into the vein of the following person, who is thus inoculated with the virus of AIDS, hepatitis and other illnesses.

There are various different single-use syringes of this type registered, such as those according to Spanish utility models 9100817 and 8801915, and US Patent No. 91US-714431, in all of which the safety device is broken by deliberate excess pressure exerted by the user of the syringe.

In short, all known models of single-use type syringes are rendered useless for further service by means of locking, which, in order to be released, must be deliberately destroyed by excess pressure applied to the syringe.

This additional pressure is only exerted at the wish of the user, who must take deliberate action, since normally, all manoeuvres in order to carry out any operation take place instinctively, with the least possible force.

Consequently, breakage by means of deliberate excess pressure on the plunger at the end of its descending path does not take place automatically, but owing to effort by the user.

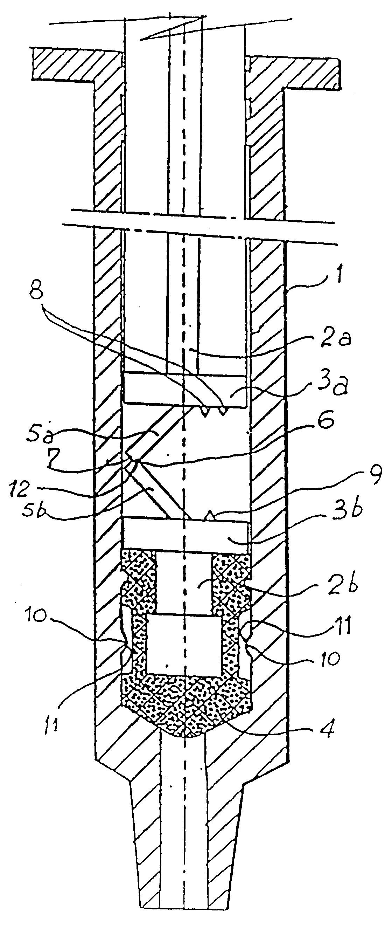

In order to prevent the breakage of the parts of the syringe from necessitating effort by the user, patent US-A-4 863 427 was produced, the four separate structural embodiments of which involve simply preventing a syringe from being re-used. No account is taken of the beneficial effect of bubbling of the fluid when it is mixed with the blood inside the body of the syringe. In the embodiment shown in figures 1, 2 and 3 of patent US-A-4 863 427, which is different from the other three separate embodiments of US-A-4 863 427, the connection strip of the two ends of the divided plunger of the syringe is in the form of a tranverse line of breakage of a strip (not a hinge), which breakage occurs outright in the plunger when its insertion through the cylinder of the syringe is begun. Owing to this thrust force to which the plunger is subjected, when the transverse weakening line is forced, the two inner ends of the divided plunger are broken away from one another.

The object of the invention is to avoid this breakage by means of excess pressure, which is disadvantageous for the user when using the safety syringe, and dangerous for the patient whilst the needle is still inserted.

Thanks to the new syringe, which can also be used once only, it is not necessary to apply any excess pressure, since the safety device breaks automatically, without any effort by the user, owing to fatigue of the material of the membrane of an intermediate, articulated tension member, by means of unhinging of two sections which constitute the operational safety component for the composite piston of the syringe, during the stage of hypodermic injection, which safety component does not break, does not shatter, does not burst and does not tear off because of effort by the user of the new syringe, but it is fatigue of the material itself of the articulated connection of the two connection sections of the composite piston of the syringe, which, without any excess of pressure during handling of the syringe, and with only the movements of opening and closing on the point of articulation, becomes fatigued and cedes when the hinge is activated, thus automatically separating the two sections of the articulated connected tension member of the composite plunger.

Consequently, the force exerted by the user of the syringe is the same as that applied in the case of a normal syringe, without a safety device for single use, since the syringe automatically becomes unusable by self-destruction after a single use, by means of fatigue, which prevents the same syringe from being used subsequently, as this could be disastrous for the second person to be treated.

Additionally, and for the above-described reason, although the syringe which is the subject of the new application is of the single-use type, it permits the advantageous, desirable bubbling of a little blood mixed with the fluid contained in the syringe, several times in succession during a single injection of a serum to be injected.

For the purpose of correct interpretation, and by way of non-limiting example, hereinafter there is described a practical embodiment of a syringe according to the invention, accompanied by a page of drawings in which:
the figure represents schematically and in vertical cross section the new syringe, inside which there is disposed the composite piston, of which the upper part corresponding to the head is disposed at the end of the descending path.

The upper of the two parts which form the composite plunger is at the begining of the separation stage, constituting the plunger, there being partially opened out the two sections of the intermediate articulated tension member which is connected to the two co-axial discs which are separated on either side of the plunger.

The upper part of the body of the syringe and of the likewise upper section of the composite plunger, is shortened.

The new hypodermic syringe is of the type which is only used once, in which, in order to complete final pumping of the piston during the use by the user, the latter deliberately destroys the syringe by splitting, brekage, bursting, tearing apart etc., by deliberate, forced increased pressure by the user of the syringe on completion of the single use, and during descent of the plunger towards the limit of the path.

The new syringe has the innovation that the deliberate breakage by excess pressure of the connection between the shaft of the plunger and its head, is replaced by automatic separation, irrespective of the will of the user, determined by fatigue of the material of the point of articulation, which is weakened during the action of pivoting of the tension member which temporarily connects the two parts which form the composite plunger.

The composite plunger of the syringe 1, consisting of two separate parts 2a and 2b which are co-axial, 2a being the part of which the shaft can partially rise through the rear part of the syringe during use thereof, and on the inner end of this upper part 2a there is disposed a perpendicular disc 3a, there being another perpendicular disc 3b on the rear part of the head 4 of the plunger.

The two end discs 3a and 3b are separate and opposite one another.

The two discs 3a and 3b of the two separate parts 2a and 2b of the syringe are connected to one another by means of an articulated tension member, by means of the central part thereof with a membrane-hinge 12, thus providing articulation of the two sections 5a and 5b.

By this means the two separate discs 3a and 3b are connected temporarily by the tension member-hinge, which at the end of one of its sections is connected to the centre of one of the two discs, and at the end of the other section of the tension member is connected to the centre of the opposite disc, there being provided throughout the point of articulation 6 a perpendicular notch 7 of a greater or lesser depth, in order to create on the side opposite that in which the notch is provided, the flexible membrane 12, which is more or less thin according to the material which constitutes it, and the number of consecutive openings and closings needed to resist the number of piston strokes of the plunger, in order to obtain bubbling in a single use of the syringe, such that rupture of the weakened connection of the flexible membrane 12 constituted by the two sections 5a and 5b of the tension member, is obtained automatically and irrespective of the will of the user of the syringe, by fatigue of the material of the flexible connection membrane 12, without additional destructive pressure exerted by the user of the syringe, thus enabling the bubbling operation to be carried out before separation takes place. By this means, until the connecting tension member is separated, the two separate discs 3a and 3b are involved in the bubbling movements which affect both sections 5a and 5b which the articulated intermediate tension member constitutes, and the tension member is sufficiently resistant to be able to remain erect, despite the pressure of the upper disc against the lower disc, or traction of the upper disc exerted on the lower disc.

However, in this type of divided plunger, in addition to the disadvantages described, there is the problem that one disc can rotate more than the other and thus create torque which could give rise to rupture by torsion of the strip-hinge, before the occurrence of separation fatigue of the two parts of the hinge which connects these composite pistons.

In order to avoid this negative torque, between the two inner ends of the piston, in the opposite surfaces of the two ends which are separated in the rest position, there are disposed three projecting lugs 8 and 9, which are aligned with one another, and which, when the composition punger is at the end of its descending path, engage in an anti-rotatory manner one above the other, at the begining of the ascending path of the composite plunger.

If it is necessary to obtain additional engagement of the head 4 of the plunger of the piston before the begining of use of the latter, in the inner circular wall of the cylinder of the syringe 1 there are disposed rigid, diametrically opposed projections 10, which correspond to recesses 11 provided in the said head 4, which projections 10 are anchored in the recesses 11 at the time of production, thus immobilising the piston such that before use it is impossible for accidental, unnecessary piston strokes to take place, which before use could give rise to fatigue of the material 12 which acts as a hinge between the two sections of the connection tension member.

It is understood that in the present case, any details of design and finish which do not alter, change or modify the essence of the invention, can be varied.

## Claims

1. New, single-use hypodermic syringe of the type in which the safety device is destroyed during use, at the moment when the piston stroke takes place, owing to deliberate excess pressure by the user, with a tension member which connects the two co-axial parts which form the plunger, and a transverse line of outright breakage at the begining of the descent of the plunger when the patient is injected, characterised in that transversely, in the manner of a hinge, there is provided an articulation vertex, the material of the tension member-hinge being suitable for each section to be maintained erect during use, and for the transverse articulation vertex to maintain its amplitude and depth, whilst acting as a hinge, in accordance with the type of material and piston strokes previously calculated as necessary in order to withstand gradual destruction during bubbling, such as to resist the plunger during a single use, and which, by means of its opening and closing flexure, moves one piston away from or towards the other, and additionally becomes fatigued, such that destruction of the tension member-hinge is gradual and progresssive, and thus destruction of the syringe is gradual, since the material of the membrane cedes gradually and progressively by fatigue, until the two sections of the tension member-hinge separate.

2. New, single-use hypodermic syringe according to the preceding Claim, in which in the opposite surfaces of the two ends which are separated in the rest position, there are disposed three opposite lugs, two in one end and a third in the opposite end, which project, and connect in line with one another, and when the composite plunger is at the limit of its descending path, these three lugs engage with one another in an anti-rotatory manner.
